# EUROPEAN PATENT SPECIFICATION

(11) **EP 3 906 095 B1**
(45) Date of publication and mention of the grant of the patent: **07.06.2023**
(21) Application number: 19713178.2
(22) Date of filing: 04.01.2019
(51) Int. Cl.: A61N 5/06, A61C 19/06, A61N 5/073

(54) **POLARIZED LIGHT EMITTING MEDICAL DEVICE, IN PARTICULAR FOR THE PREVENTION AND TREATMENT OF GINGIVAL AND PERIODONTAL DISEASES**
MEDIZINISCHE VORRICHTUNG MIT POLARISIERTEM LICHT, INSBESONDERE ZUR VORBEUGUNG UND BEHANDLUNG VON ZAHNFLEISCH- UND PARODONTALERKRANKUNGEN
DISPOSITIF MÉDICAL ÉMETTEUR DE LUMIÈRE POLARISÉE, SERVANT EN PARTICULIER À LA PRÉVENTION ET AU TRAITEMENT DE MALADIES GINGIVALES ET PARODONTALES

(43) Date of publication of application: 10.11.2021
(73) Proprietor: FENYO, Márta Katalin, 2000 Szentendre (HU); Zsigmond, Tomás, 930 38 Nový Zivot (SK)
(72) Inventor: FENYO, Márta Katalin, 2000 Szentendre (HU); Zsigmond, Tomás, 930 38 Nový Zivot (SK)
(74) Representative: Danubia Patent & Law Office LLC
(86) International application number: PCT/HU2019/050001
(87) International publication number: WO 2020/141337

(56) References cited:
- WO-A2-2006/103678
- US-A1- 2006 047 329
- US-A1- 2007 219 600

## Description

### FIELD OF THE INVENTION

The invention relates to a polarized light emitting medical device, in particular for the prevention and treatment of gingival (gums) and periodontal diseases, in particular e. g. for the prevention and treatment of parodontitis, by means of which, in case of an inflammation in the oral cavity the affected tissues can be effectively treated, and, respectively, by regular use of the device the development of an inflammatory condition can be prevented, by taking advantage of the healing effect of the polarized light.

### BACKGROUND OF THE INVENTION

It is scientifically proven that polarized light therapy with its biological stimulating feature can be efficiently utilized for defence against pathogenic bacteria (see: A. Falus, M. Fenyő, K. Éder., A. Madarasi: "A polarizált fény epigenetikai tényezői a gyulladásgátlásban" ['Epigenetic Factors of Polarized Light in Anti-Inflammation Therapy'] - Orvosi Hetilap ['Medical Weekly'] No. 27 of 2011/152, pp. 1492-1499).

Polarized light therapy achieves destruction of pathogens through biostimulation, by mobilizing the natural defence mechanism of the body, see. M. Fenyő: Theoretical and experimental basis of biostimulation - Optics and Laser Technology, August 1984, https://pdfs.semanticscholar.org/d9c7/432a07f15592261c46dfd4c5b26a04e457a3.pdf Hungarian patent HU 186.081 discloses an apparatus for stimulating biological processes associated with cellular activity, in particular for inspiring the healing of pathological formulations such as wounds, ulcers and other injuries on the body surface, the apparatus comprising a polarized light emitting optical arrangement having an incoherent light source featuring a wavelength over 300 nm, and a polarizer unit integrated with or separate from said light source, and a light guide system for directing the light beams in a given direction. The polarizer of the apparatus is optionally a polar filter. The light source and the optical arrangement including the polarizer are arranged in a cylindrical housing.

Hungarian patent HU 191.836 discloses a light therapy device, particularly for wound healing, comprising a power supply, connected to the power supply a light source emitting polarized light or equipped with a polarizer, and preferably an optical device influencing the light flow emitted from the light source, wherein the light source is comprised of one or more light emitting diodes of small spectral bandwidth preferably in a matrix-like arrangement, the light emitting diodes operating in the infrared range. The power supply comprises a modulator for pulse modulation of the current of the light emitting diode.

In a publication entitled "Adjunctive dental therapy via tooth plaque reduction and gingivitis treatment by blue light-emitting diodes tooth brushing" published in the December 2015 issue of the Journal of Biomedical Optics - - authors: Elina Genina, Vladimir Titorenko, Andrey Belikov, Alexey Bashkatov, Valery Tuchin; see at https://www.spiedigitallibrary.org/journals/journal-of-biomedical-optics/volume-20/issue-12?SSO=1#SpecialSectiononOpticalMedicalImagingStandard - an adjunctive dental therapy is disclosed, wherein a combination of mechanical/chemical means of plaque elimination with photodynamic antibacterial therapy is proposed for more efficient reduction of dental plaques and more efficient treatment of gingival inflammation, in a way that simultaneously with mechanic cleaning provided by toothbrush bristles, the target surface e. g. the gum to be treated is also illuminated with blue LEDs. The proposed device comprises a toothbrush having light source composed of LED inside its head portion to emit a blue light parallel to the longitudinal direction of the bristles of the toothbrush, the blue light featuring a wavelength in the range of 405 to 420 nm and a power density of 2 mW/cm².

A light therapy medical device marketed by the company Zepter known as 'BIOPTRON Medolight'- see at https://www.zeptermagazin.hu/shop/zepter-medical/572/ - has a housing equipped with control buttons for handling the device. According to its product description, the device comprises a number of light-emitting diodes that generate waves featuring the biologically most effective red and closely infrared light ranges (640 nm and 880 nm, respectively). At these wavelengths, the light has a blood circulation improving, pain reducing and anti-inflammatory biostimulation effect, also expediting wound healing. The fields of application of the device include the treatment of gingivitis as well.

Further, document WO 2006 103 678 discloses a device for treatment of body cavities, in particular for gums and periodontal diseases. The device comprises an array of light sources that can emit polarized light.

It is a drawback of the known devices is that they are not suitable for an effective light treatment of the oral cavity, as they do not provide, or just circuitously or inefficiently provide illumination of the internal parts of the oral cavity, in particular the hardly accessible surface areas of the gums with polarized light.

The object of the invention is to make use of the biological stimulation potency of polarized light in preventing inflammatory diseases and in efficient treatment of diseases evolved in the oral cavity, by means of a medical device adapted for such a purpose.

### SUMMARY OF THE INVENTION

To achieve this objective, there has been developed a polarized light emitting medical device with a housing formed as a handle of the medical device, in which there is an electronic control unit which is control-signal connected with a polarized light emitting arrangement, said polarized light emitting arrangement comprising at least one light source connected with said electronic control unit and a polarizer optical unit optically connected with said light source, for forming a polarized light beam from the light rays emitted by said light source, wherein, the medical device according to the invention comprises at least one polarized light emitting treatment console connected to said housing, and incorporating said light source and said polarizer optical unit optically connected therewith.

In the context of the present invention, the feature that the housing is formed as a handle of the medical device shall be interpreted that it is a housing which can be comfortably held in one hand, having an elongated, typically but not necessarily cylindrical form, dimensioned preferably between 5 cm and 25 cm in length and weighting preferably not more than 500 g and having sufficient firmness, similar to which being known as housings of electric toothbrushes, mouth showers or flashlights. Also said polarizer optical unit of said polarized light emitting arrangement is feasible in a manner known per se, preferably with a polarizer filter (actually with a polarizing foil applied on a plexi carrier) positioned in front of the light source.

There are two tentacle-shaped polarized light emitting treatment consoles each linked to the opposite ends of said housing, wherein both tentacle-shaped polarized light emitting treatment consoles are of different geometrical design. Said tentacle-shaped polarized light emitting treatment consoles are, due to their different geometrical designs, convenient for reaching different target surface areas, resulting in that polarized light can be more precisely directed to a particular surface area inside the oral cavity by using the light emitting treatment console which is more appropriate for reaching that particular surface area.

The one tentacle-shaped polarized light emitting treatment console connected to the housing has a geometrical design of being flexed inwards at an angle within a range of 15° to 60° in the direction of the emission of the polarized light relative to the longitudinal axis of the housing, while the other tentacle-shaped polarized light emitting treatment console connected to the housing has a geometrical design of being flexed inwards at an angle within a range of 90° to 135° in the direction of the emission of the polarized light relative to the longitudinal axis of the housing.

The light emitting treatment console being flexed at a smaller angle inwards in the direction of the emission of the polarized light is primarily suitable for treating the surface areas of the gums facing outwards - towards the vestibulum oris and the cheeks, respectively -, while the light emitting treatment console flexed at a greater angle inwards in the direction of the emission of the polarized light is primarily suitable for reaching and illuminating the hind surface areas of the oral cavity and the inward facing surface areas of the gums more efficiently by polarized light, so that the preferred embodiment provides a convenient, precise and, consequently, efficient scanning over and illuminating by polarized light most of the mucosal surfaces of the oral cavity and the outward and inward facing surface areas of the gums.

Preferably, the tentacle-shaped socket/s/ of said treatment consoles form a monolithic structural piece with the housing.

In a further preferred embodiment of the above medical device of the invention, said at least one polarized light emitting treatment console has a retentively bendable tentacle-shaped socket. In case of this embodiment the tentacle-shaped socket of the polarized light emitting treatment console can be bent at an angle and/or curved in an arc to take a shape which enables positioning the treatment console within the oral cavity so that the polarized light emitted by the treatment console can be most efficiently directed to a target surface area to be illuminated during a particular treatment. Efficiency in this respect means for example that the angle of incidence of the polarized light is perpendicular to the target surface, and/or the polarization of the polarized light is perpendicular to the longitudinal direction of a wound to be treated. The adjustability of the above described preferred embodiments of the medical device of the invention to particular requirements results not only in more precise positioning of the light emitting treatment console to the target surface area to be treated, but also in that the professional carrying out a treatment or even the patient operating the device himself can conveniently hold the device so that the polarized light emitting treatment console is positioned in a proper position, which ultimately supports the efficiency of the treatment.

The adjustability of the medical device of the invention to particular requirements can be also achieved by another preferred embodiment in which at least one polarized light emitting treatment console is an interchangeable component linked by a releasable connection, e. g. by bayonet lock to the housing. In case of this embodiment, there may be provided different tentacle-shaped polarized light emitting treatment consoles of different shape designs and different sizes, featuring different angles of deflection in the direction of the emission of the polarized light relative to the longitudinal axis of the housing, to suit different treatment needs, so that a right suitable interchangeable treatment console can be applied to a particular treatment.

Said housing formed as a handle of the medical device may also be equipped with an electric power input and an electronic connector input for the connection of an external device - for example a personal computer - with said electronic control unit, and it is also equipped with external operable control buttons which are connected with said electronic control unit.

Said electric power input and said electronic connector input provided for electronic signal connection can be realized by a single complex connection socket, e. g. by a combined USB connector socket. By means of said external device cooperating with said electronic control unit, the treatment parameters, in particular the intensity, the light output of the light sources, the color, and the color temperature of the polarized light emitted corresponding to the patient and the treatment purpose, and the set parameters can be eventually stored.

According to the clinical experiences of test treatments carried out with a medical device of the invention, it is expedient to set the light output of the light source preferably in the range of 40 to 140 mW/cm², depending on the particular treatment purpose. With such a light output density, an effective energy density can be achieved on the treated tissue surface during an average treatment period of 2 to 20 minutes. And the wavelength range of the emitted light shall be expediently set within the ranges of the visible light wavelength and the closely infrared wavelength, preferably in the range of 380 nm to 860 nm, so that the diversified biostimulatory effects of the light components of different wavelengths of said range may prevail.

Said electronic control unit may be equipped with a short-range wireless communication module for control-signal and data transfer, such as e. g. a Bluetooth device.

In a further preferred embodiment of the medical device of the invention, said housing formed as a handle of the medical device is equipped with external operable control buttons which are connected with said electronic control unit. Said control buttons serve for switching the medical device on and off, in case of a device having two polarized light emitting treatment consoles for choosing and operating the polarized light emitting treatment console to be actually used.

in a further preferred embodiment of the medical device of the invention, said light source or light sources, respectively, is/are constituted by halogen light source/s/ and/or light emitting diodes (LEDs).

In a further preferred embodiment of the medical device of the invention, said light source or light sources, respectively, is/are light sources controllable by the electronic control unit within a light output range and a wavelength range. Such an embodiment enables adjusting the appropriate intensity and wavelength of the polarized light to the requirements of a particular treatment and exploiting the various physiological effects of various color and color temperature components of polarized light for the specific treatment purpose. For example, the polarized light of blue color range prevail a favourable biological stimulating effect for the contraction of the vessels and the relaxation of the swollen mycoderm. The components of the shorter wavelength ranges are further advantageous in strengthening the vascular walls and also in the reepithelialisation of damaged mycoderm. On the other hand, the light components of longer wavelength such as those of the red and infrared range have a more beneficial effect on dilating blood vessels and inducing circulation-stimulating effects. Compared to the shorter wavelength components, the light components of the red wavelength range of the electromagnetic spectrum penetrate deeper into the treated tissue, for which reason the use of polarized light of the warm white wavelength range is generally preferred.

In a further preferred embodiment at least one of said light sources comprises at least one LED for emitting warm-white color temperature light, preferably of the wavelength range of 380 to 800 nm, and at least one LED for emitting infrared light, preferably of the wavelength range of 800 to 860 nm. The clinical experiences of test treatments carried out with a medical device of the invention have shown that the polarized light of a warm white color temperature wavelength range improves the effectiveness of the polarized light treatments in the tissue stock closer to the mucosal surface, while the polarized light of the infrared light range improves the effectiveness of the treatments in the tissue stock located deeper relative to the mucosal surface, even in bony tissues.

According to our experiences, in case of the above preferred embodiment of the medical device of the invention it is a particularly advantageous embodiment where the light output density of the LED for emitting warm-white color temperature light is two to five times higher than the light output density of the LED for emitting infrared light. So for example, the light output density of the warm-white color temperature light emitting LED is 50 to 75 mW/cm², while the light output density of the infrared light emitting LED is 15 to 25 mW/cm².

In a further preferred embodiment of the medical device of the invention, said electronic control unit and a driving circuit of said polarized light emitting arrangement are fitted on a printed circuit board.

Further, in a preferred embodiment of the invention, said electronic control unit comprises a control panel or control panels, respectively, for light source control.

In a further preferred embodiment of the medical device of the invention, said light source control panel or panels being signal-connected with the electronic control unit are inserted in said polarized light emitting treatment consoles.

In a further preferred embodiment of the medical device of the invention, said polarized light emitting treatment console or consoles, respectively, are equipped with an overheating sensor being signal-connected with the electronic control unit. In case a light source is overheated, in response to a warning signal provided by the temperature sensor the electronic control unit will reduce the light output of the light source, or switches off the operation of the device where appropriate. This is necessary since the LEDs and the polarizing filters are also rather sensitive to overheating, and the overheating of a treatment console could even endanger the comfort and safety of a treatment.

Additionally or alternatively to the two-tentacle design, said polarized light emitting treatment console or consoles, respectively, feature an oval window formed for said polarizer optical unit at the distal end portion of said polarized light emitting treatment consoles, wherein the longer axis of said oval window is parallel to the longitudinal direction of the respective polarized light emitting console, and said polarizer optical unit is positioned in said oval window to define the direction of polarization parallel to the longer axis of the oval window.

This embodiment is particularly advantageous for the treatment of elongate surgical incisions and other longitudinal injuries and scars, respectively. According to the clinical experiences of test treatments carried out with a medical device of the invention, such wounds heal faster and nicer if the direction of polarization is perpendicular to the longitudinal direction of the wound. In case of dental implantation and several other oral surgical interventions, the surgical incisions run parallel to the arch of the jaw and the denture. With this preferred embodiment of the medical device of the invention, a professional carrying out a treatment can hold the device in an obviously handy manner so that the longer axis of said oval window of a polarized light emitting treatment console, and consequently the direction of polarization is just perpendicular to the line of the surgical wound, which results in higher efficiency of treatment.

An outstanding advantage of the medical device according to the invention is that it enables scanning over the inner surfaces of the oral cavity and accurate illumination of the target tissue surfaces by polarized light, in a way that is even convenient for the person performing the treatment, which enhances both the healing and the anti-inflammatory efficiency of the treatment. A further advantageous effect of the invention is that it enables polarized light treatment of even those surface areas of the oral cavity which are the hardly or non-accessible by means of known devices. Therefore, by use of the device of the invention significant results can be obtained in the treatment of inflammatory diseases of the gums and the periodontal, in the prevention of the development of such conditions and in inspiring wound healing. Due to the provision of destroying pathological bacteria in pouches developed due to inflammation by way of an effective treatment with polarized light, the medical device of the invention provides an efficient protection against parodontitis.

Further, the medical device of the invention can be effectively used also for symptomatic treatment, namely, the pain caused by inflammation can be alleviated, and gum bleeds can be suppressed or stopped. By regular treatments by polarized light with the medical device of the invention, periodontitis and the development of inflammatory conditions in general can be prevented, and the progress of gingivitis can be stopped and even reversed.

By using the medical device of the invention, all the above mentioned beneficial effects are achieved by local biological stimulation of the human organism, stimulating the locally acting natural defence mechanisms, stimulating the body's own regeneration processes, for example by expediting epithelisation, by regenerating the capillary vessels and the mycoderm, with no external chemical effects and harmful side effects.

Further possible uses of the medical device of the present invention include - subject to suitable geometric design of the tentacle-shaped polarized light emitting treatment console - anti-inflammatory, wound healing and inflammation preventing biological stimulating treatments of other body cavities, e. g. for treating the nasal mycoderm or the auditory canal and the eardrum, as well as gynecological application of the device with the same intent. For the treatment of the auditory canal or the nose inner surface area, the polarized light emitting treatment console shall be designed to emit polarized light radially outwards, a possible realization of which is for example a linear LED arrangement ensphered with a cylindrical polarizing foil in a manner that that the direction of polarization is parallel to the generatrix of the cylinder formed by the polarizing foil.

### BRIEF DESCRIPTION OF THE DRAWINGS

The invention will now be described in more details with respect to the drawings illustrating some preferred embodiments of the invention. In the drawings:
Figure 1 shows a schematic side-sectional view of an embodiment of a medical device of the disclosure;
Figure 2 shows a schematic side-sectional view of another preferred embodiment of a medical device of the invention;
Figure 3A illustrates a view of a preferred embodiment of a medical device of the invention wherein a half part of the device housing is invisible/removed to illustrate the inside of the device;
Figure 3B shows a perspective view of the preferred embodiment of Fig. 3A;
Figure 4 shows a front view of a part of a preferred embodiment of a polarized light emitting treatment console of a medical device of the invention;
Figure 5 illustrates a schematic view of a preferred embodiment of a medical device of the invention positioned to a target surface of treatment in the oral cavity.

### DETAILED DESCRIPTION OF ILLUSTRATED EMBODIMENTS

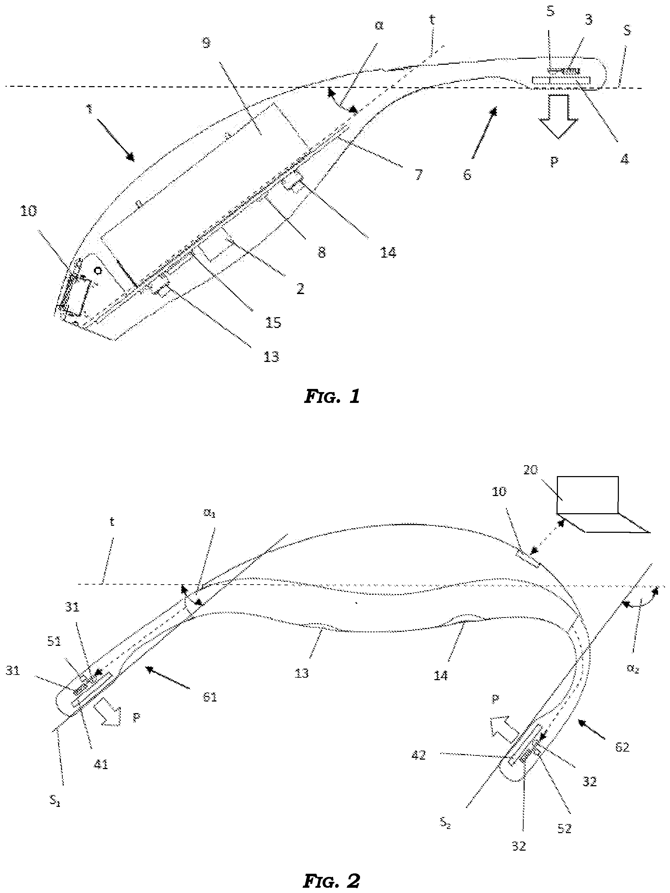

An example of a medical device as shown in Fig. 1, comprises a housing 1 and a polarized light emitting treatment console 6 connected thereto. The housing 1 is dimensioned and designed to also form a handle of the medical device. The polarized light emitting treatment console 6 has a tentacle-like flexed geometric design narrowing from the housing 1 to the distal end portion of the polarized light emitting treatment console 6, and inwards flexed in the direction of the emission of polarized light P relative to the longitudinal axis t of the housing 1. A light source 3 and a polarizer optical unit 4 of a polarized light emitting arrangement are positioned inside said distal end portion of the polarized light emitting treatment console 6. Adjacent to said light source 3 - in this particular case a LED layout - there is positioned a temperature sensor 5 which is signal-connected with an electronic control unit 2. In case the light source 3 is overheated, the temperature sensor 5 sends a warning signal to the electronic control unit 2 which reduces the light output of the light source 3. In case the overheating exceeds a set threshold, the electronic control unit 2 switches off the device. In a particular case, the tentacle-shaped polarized light emitting treatment console 6 may be formed as an interchangeable individual component which is linked in a releasable manner to the housing 1.

A battery 9, preferably a rechargeable battery and the electronic control unit 2 (preferably a processor unit comprising a RAM memory) fitted on a printed circuit board 7, and a driving circuit 8 of said polarized light emitting arrangement are arranged inside the housing 1. Said driving circuit 8 of the polarized light emitting arrangement is control-signal connected with the electronic control unit 2. Control buttons 13 and 14, and a short-range wireless communication module 15 for control-signal communication and data transfer - in a particular case a Bluetooth device - which are signal-connected with said electronic control unit 2, are also mounted on said printed circuit board 7. Said control buttons 13 and 14 may serve for switching the medical device on and off by an operator and, in a particular case, for adjusting the intensity and/or the wavelength of the polarized light emitted by the polarized light emitting treatment console 6. Said wireless communication module 15 for control-signal communication and data transfer may provide signal connection between the electronic control unit 2 and an external device such as a data storage device or a personal computer.

The housing 1 is further equipped with a connector input 10, in a particular case an USB connector input, adapted for electric power supply of the device from an external power source, connecting the battery 9 on a charger and, if necessary, enabling a wired connection of the medical device of the invention with an external device such as a data storage device or a personal computer.

The tentacle-shaped polarized light emitting treatment console 6 shown in Fig. 1 is flexed inwards, i. e. in the direction of the emission of polarized light P, relative to the longitudinal axis t of the housing 1. The degree of arcuate deflection of the tentacle-shaped polarized light emitting treatment console 6 relative to the longitudinal axis t of the housing 1 provides an angle α between plane S of the polarizer optical unit 4 in the distal end portion of the polarized light emitting treatment console 6 and the longitudinal axis t of the housing 1 ranging around 35° to 40°. The degree of arcuate deflection of the tentacle-shaped polarized light emitting treatment console 6 relative to the longitudinal axis t of the housing substantially determinates which target areas in the oral cavity can be accessed and illuminated by polarized light in an optimal way. A polarized light emitting treatment console 6 with a smaller arc of deflection inwards, where the angle α ranges 0° to 90°, and preferably 15° to 60° within this range, is rather suitable for treating by polarized light the target surface areas of the oral cavity close to the orifice and the surface areas of the gums facing outwards (towards the vestibulum oris and cheeks), while a polarized light emitting treatment console 6 which is backward curved at an angle α ranging 90° to 170°, and preferably 90° to 135° within this range, is rather suitable for scanning and illuminating/treating the surface areas of the oral cavity facing towards the pharynx and the inward facing surface areas of the gums.

The proper shape of the polarized light emitting treatment console 6 is also important from the point of view that the person operating the device according to the invention can drive and hold the device with a natural hand position during a treatment.

In a preferred embodiment of the medical device of the invention illustrated in Fig. 2, a housing 1 which also forms a handle of the medical device, is connected at its one end - the left-side end in Fig. 2 - with a polarized light emitting treatment console 61 and connected at its opposite end - the right-side end in Fig. 2 - with a polarized light emitting treatment console 62, wherein said polarized light emitting treatment console 61 is flexed inwards, i. e. in the direction of the emission of polarized light P at a relatively low angle relative to the longitudinal axis t of the housing 1, while said other polarized light emitting treatment console 62 is flexed inwards at a relatively greater angle - in the particular case curved backward - relative to the longitudinal axis t of the housing 1. A light source 31 and a polarizer optical unit 41 of a polarized light emitting arrangement are positioned inside said distal end portion of the polarized light emitting treatment console 61. Adjacent to the light source 31 - in the particular case a LED layout comprising several light emitting diodes - there is positioned a temperature sensor 51 which is signal-connected with an electronic control unit 2. A light source 32 and a polarizer optical unit 42 of a polarized light emitting arrangement are positioned inside said distal end portion of the polarized light emitting treatment console 62. Adjacent to said light source 32 - in the particular case a LED layout comprising several light emitting diodes - there is positioned a temperature sensor 52 also signal-connected with an electronic control unit 2.

Plane S₁ of the polarizer optical unit 41 is at an angle α₁ to the longitudinal axis t of the housing 1, while plane S₂ of the polarizer optical unit 42 is at angle α₂ to the longitudinal axis t of the housing 1, wherein α₁ is in the range of around 15° to 60° and angle α₂ is in the range of around 90° to 135°. In case of this preferable embodiment of the medical device of the invention, the light emitting treatment console 61 is primarily suitable for treating the surface areas of the gums facing outwards by polarized light in a handy and efficient manner, while the light emitting treatment console 62 of backward curved shape is primarily suitable for reaching and illuminating by polarized light the hind surface areas of the oral cavity and the inward facing surface areas of the gums in an advantageous manner.

The housing 1 is equipped with control buttons 13 and 14 adapted for switching the medical device on and off and for operating the polarized light emitting treatment consoles 61 and 62 by an operator. The housing 1 is further equipped with a connector input 10 through which said electronic control unit 2 of the medical device is connectable with an external device, a personal computer 20 in the particular embodiment.

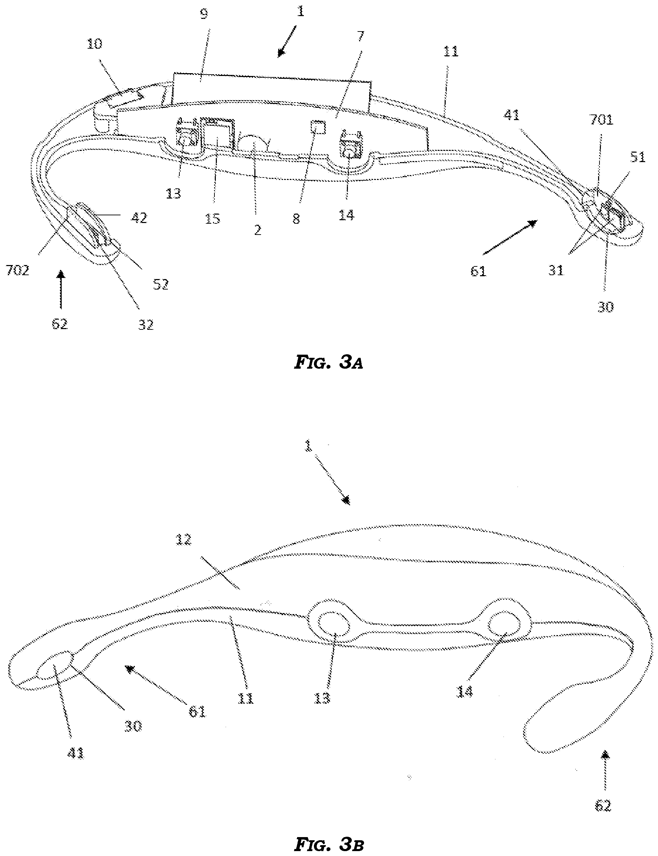

Figure 3A illustrates more in detail the internal structure of an embodiment of a medical device of the invention having two polarized light emitting treatment consoles 61 and 62. In this embodiment, the housing 1 and the tentacle-shaped sockets of the polarized light emitting treatment consoles 61 and 62 are composed of two socket pieces fitted together along a longitudinal bisecting plane. Fig. 3A shows only socket piece 11 as the other piece (socket piece 12, see in Fig. 3B) is invisible in Fig. 3A so that the internal structure of the embodiment of the invention can be illustrated in the drawing. Socket pieces 11 and 12 form a monolithic structural piece accomplishing the housing 1 and the tentacle-shaped sockets of the polarized light emitting treatment consoles 61 and 62.

Figs. 3A and 3B illustrate that the polarized light emitting treatment console 61 protruding from the housing 1 in the one direction - to the right in Fig.3A - is flexed inwards, i. e. in the direction of the emission of polarized light P at a relatively low angle relative to the longitudinal axis t of the housing 1, while the polarized light emitting treatment console 62 protruding from the housing 1 in the opposite direction - to the left in Fig.3A - is curved backward at a relatively greater angle. The light source 31 (constituted preferably by LEDs) of the polarized light emitting arrangement are set inside the distal end portion of the polarized light emitting treatment console 61, with the polarizer optical unit 41 being positioned in front of the light source 31. Said polarizer optical unit 41 (marked with a dotted line so that the rest of the adjacent parts are visible in the illustration) is a polarizer foil applied on an oval plexi carrier. Said light sources 31 and a temperature sensor 51 positioned in their close proximity are mounted on a light source control panel 701 placed inside the distal end portion of the polarized light emitting treatment console 61, and signal-connected with said electric control unit 2. In front of the polarizer optical unit 41 in the direction of the emission of polarized light P there is an oval window 30 shaped conform to the polarizer optical unit 41. Said oval window is an oval resection cut out of the socket of the light emitting treatment console 61, in the particular case of the respective distal portions of the socket pieces 11 and 12. A half-oval cut out section of the oval window 30 is shown in Fig. 3A.

On the opposite side, the light sources 32 (constituted preferably by LEDs) of the polarized light emitting arrangement are set inside the distal end portion of the polarized light emitting treatment console 62, with the polarizer optical unit 42, actually a polarizer foil applied on an oval plexi carrier being positioned in front of the light source 32. Said light source 32 and a temperature sensor 52 positioned in its close proximity are mounted on a light source control panel 702 placed inside the distal end portion of the polarized light emitting treatment console 62, and signal-connected with said electric control unit 2. In front of the polarizer optical unit 42 in the direction of the emission of polarized light P there is an oval window not shown in Fig. 3A, shaped the same manner as the oval window 30 of the polarized light emitting treatment console 61 described above.

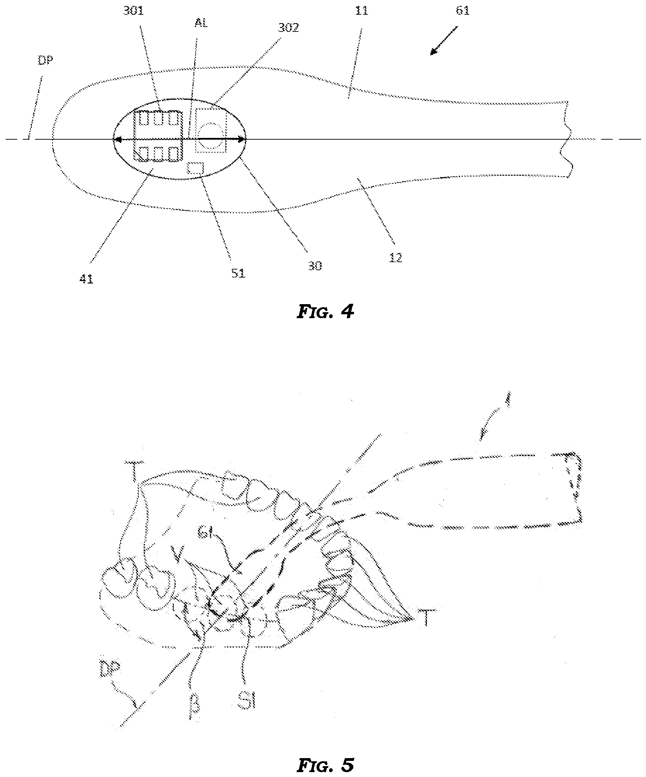

In the housing 1 of the embodiment of the invention shown in Figs. 3 and 4 - likewise the embodiment illustrated in Fig. 1 - there is a battery 9, preferably a rechargeable battery and a printed circuit board 7. The electronic control unit 2 (actually a processor comprising a RAM memory), the driving circuit 8 of said polarized light emitting arrangement signal-connected with said electronic control unit 2, mounted on the printed circuit board 7, control buttons 13 and 14, and a short-range wireless communication module 15 for control-signal communication and data transfer - in a particular case a Bluetooth device - which are signal-connected with said electronic control unit 2, are mounted on said printed circuit board 7. Said control buttons 13 and 14 may serve for switching the medical device on and off by an operator, for selecting which of the polarized light emitting treatment consoles 61 or 62 shall actualy be operable and, in a particular case, for adjusting the intensity and/or the wavelength of the polarized light emitted by the polarized light emitting treatment consoles 61 and 62. By means of said wireless communication module 15 for control-signal communication and data transfer, signal connection can be achieved between the electronic control unit 2 and an external device such as a data storage device or a personal computer.

The operation parameters of the medical device of the invention, even patient-specific treatment parameters in a particular case set by electronic control unit 2 can be stored in the RAM memory of the electronic control unit 2 and/or in external data storage devices or personal computers or in memories of other suitable mobile devices.

In Fig. 3B the embodiment of the medical device of the invention described with respect of Fig. 3A is illustrated in a perspective view. As shown in Fig. 3B, the medical device housing 1 and the tentacle-shaped sockets of the polarized light emitting treatment consoles 61 and 62 protruding from said housing 1 in opposite directions are constituted by two socket pieces 11 and 12 fitted together along a longitudinal bisecting plane, so that the housing 1 and the sockets of the polarized light emitting treatment consoles 61 and 62 form a monolithic structural piece. Fig. 3B also illustrates a preferable positioning of the control buttons 13 and 14 on the inner side of the housing 1. The oval window 30 cut out of the socket pieces 11 and 12 at the distal end portion of the polarized light emitting treatment console 61 is positioned inwards (in the direction of the emission of polarized light) in front of the polarizer optical unit 41.

Fig. 4 shows a front view of a part of a preferred embodiment of a polarized light emitting treatment console 61 of a medical device of the invention. As shown in the front view in Fig. 4, behind the oval window 30 cut off of the socket pieces 11 and 12 at the distal end portion of the polarized light emitting treatment console 61 there is a conform oval shaped polarizer optical unit 41 arranged parallel to the oval window 30. Behind the polarizer optical unit 41 there are LEDs 301 and 302, and the temperature sensor 51, wherein LEDs 301 and 302 constitute the light source 31 of the polarized light emitting treatment console 61. In a preferred embodiment of the medical device of the invention, said LED 301 is a light emitting diode adapted for emitting warm-white color temperature light, said LED 302 is a light emitting diode adapted for emitting infrared light. The LEDs 301 and 302 are preferably light sources of adjustable light output and wavelength, so that they can be controlled by the electronic control unit 2, through the light source control panel 701, within a light output range and a wavelength range, so that the light output and the wavelength of the polarized light emitted by the LEDs 301 and 302 are suitable for a particular treatment purpose. The experiences of test treatments with the device according to the invention showed that particularly favourable treatment results could be achieved by a setting wherein the light output of the warm-white color temperature light emitting LED 301 is two to five times higher - more preferably within this range three to four times higher - than that of the infrared light emitting LED 302. Consequently, if the light output of the warm-white color temperature light emitting LED 301 is set for example at a range of 50 to 75 mW/cm², the light output of the infrared light emitting LED 302 shall be preferably set at a range of around 15-25 mW/cm².

The oval window 30 of the polarized light emitting treatment console 61 is positioned so that the longer axis AL of the oval window 30 is parallel to the longitudinal dimension of the polarized light emitting treatment console 61 which direction is also coincident with a direction of polarization DP defined by the polarizer optical unit 41.

In case of the preferred embodiment as described above, the operator of the medical device of the invention conducting a treatment can conveniently hold the medical device such a manner that the longer axis AL of the oval window 30 and consequently the direction of polarization DP is parallel to a surgical incision SI inside the oral cavity as illustrated in Fig. 5. Such a positioning results in improving the effectiveness of the treatment.

Fig. 5. schematically shows a fraction of the inside of an oral cavity with some teeth T of the lower denture and vacancies V marked in dotted lines, featuring a gingival surface area affected by oral surgery. In the illustrated case, the target surface area of the treatment by polarized light is the a surgical incision SI made along the vacancies V, which target surface area shall be illuminated by polarized light with the view of preventing inflammation and stimulating the wound healing. The professional person carrying out a treatment by means of the medical device according to the invention shall approach the target area to be treated in a manner that the longitudinal dimension of the polarized light emitting treatment console 61 as well as the longer axis AL of the oval window 30 and the direction of polarization DP determined by the polarizer optical unit 41 is positioned to the direction of the surgical incision SI at a right angle β. Experimental experiences have proved that the direction of the polarization of the polarized light applied for the treatment significantly affects the efficiency of the biologic stimulation achieved, namely, the best wound healing efficiency is reached with a direction of polarization DP of the polarized light perpendicular to the line of the wound.

The invention is defined in the following claims. Other embodiments, examples etc. are not a part of the invention.

## Claims

1. A polarized light emitting medical device, in particular for the prevention and treatment of gingival and periodontal diseases, with a housing (1) formed as a handle of the medical device, in which there is an electronic control unit (2) which is control-signal connected with a polarized light emitting arrangement, said polarized light emitting arrangement comprising at least one light source (3, 31, 32) connected with said electronic control unit (2) and a polarizer optical unit (4, 41, 42) optically connected with said light source (3, 31, 32) for forming a polarized light beam (P) from the light rays emitted by said light source (3, 31, 32), said polarized light emitting arrangement comprises at least one polarized light emitting treatment console (6, 61, 62) connected to said housing (1) and incorporating said light source (3, 31, 32) and said polarizer optical unit (4, 41, 42) optically connected therewith, **characterized in that** said polarized light emitting treatment console/s/ (6, 61, 62) feature/s/ an oval window (30) formed for said polarizer optical unit (4, 41, 42) at the distal end portion of said polarized light emitting treatment console/s/ (6, 61, 62), the longer axis (AL) of said oval window (30) being parallel to the longitudinal direction of the respective polarized light emitting console (6, 61, 62), and said polarizer optical unit (4, 41, 42) is positioned in said oval window (30) to define the direction of polarization (DP) parallel to the longer axis (AL) of the oval window (30).

2. The polarized light emitting medical device of claim 1, wherein the at least one polarized light emitting treatment console (6, 61, 62) is a tentacle-shaped component, wherein said light source (3, 31, 32) and said polarizer optical unit (4, 41, 42) optically connected therewith are arranged in the distal end portion of said tentacle-shaped treatment console (6, 61, 62).

3. The polarized light emitting medical device of any of claims 1 or 2, wherein said at least one polarized light emitting treatment console (6, 61, 62) has a tentacle-like flexed geometric design, flexed inwards in the direction of the emission of polarized light (P) relative to the longitudinal axis (t) of the housing (1).

4. The polarized light emitting medical device of any of claims 2 or 3, wherein it has two tentacle-shaped polarized light emitting treatment consoles (61, 62) each linked to the opposite ends of said housing (1), wherein both tentacle-shaped polarized light emitting treatment consoles (61, 62) feature different geometrical designs.

5. The polarized light emitting medical device of claim 4, wherein the one of the tentacle-shaped polarized light emitting treatment consoles (61) connected to the housing (1) has a geometrical design of being flexed inwards at an angle (α₁) within a range of 10° to 90° in the direction of the emission of the polarized light (P) relative to the longitudinal axis (t) of the housing (1), while the other tentacle-shaped polarized light emitting treatment console (62) has a geometrical design of being flexed inwards at an angle (α₂) within a range of 90° to 170° in the direction of the emission of the polarized light (P) relative to the longitudinal axis (t) of the housing (1).

6. A polarized light emitting medical device, in particular for the prevention and treatment of gingival and periodontal diseases, with a housing (1) formed as a handle of the medical device, in which there is an electronic control unit (2) which is control-signal connected with a polarized light emitting arrangement, said polarized light emitting arrangement comprising at least one light source (31, 32) connected with said electronic control unit (2) and a polarizer optical unit (41, 42) optically connected with said light source (31, 32) for forming a polarized light beam (P) from the light rays emitted by said light source (31, 32), said polarized light emitting arrangement comprises two tentacle-shaped polarized light emitting treatment consoles (61, 62) each linked to the opposite ends of said housing (1), and incorporating said light source (31, 32) and said polarizer optical unit (41, 42) optically connected therewith, **characterized in that** the one of the tentacle-shaped polarized light emitting treatment consoles (61) connected to the housing (1) has a geometrical design of being flexed inwards at an angle (α₁) within a range of 15° to 60° in the direction of the emission of the polarized light (P) relative to the longitudinal axis (t) of the housing (1), while the other tentacle-shaped polarized light emitting treatment console (62) has a geometrical design of being flexed inwards at an angle (α₂) within a range of 90° to 135° in the direction of the emission of the polarized light (P) relative to the longitudinal axis (t) of the housing (1).

7. The polarized light emitting medical device of any of claims 1 to 6, wherein said at least one polarized light emitting treatment console (6, 61, 62) has a retentively bendable tentacle-shaped socket.

8. The polarized light emitting medical device of any of claims 1 to 7, wherein said at least one polarized light emitting treatment console (6, 61, 62) is an interchangeable component linked by a releasable connection to the housing (1).

9. The polarized light emitting medical device of any of claims 1 to 6, wherein said polarized light emitting treatment console or consoles (61, 62) has / have tentacle-shaped socket/s/ and form/s/ a monolithic structural piece with the housing (1).

10. The polarized light emitting medical device according to any of claims 1 to 9, wherein said housing (1) formed as a handle of the medical device is equipped with external operable control buttons (13, 14) which are connected with said electronic control unit (2).

11. The polarized light emitting medical device according to any of claims 1 to 10, wherein said light source or sources (3, 31, 32), respectively, is/are constituted by halogen light source/s/ and/or light emitting diodes (LEDs).

12. The polarized light emitting medical device according to any of claims 1 to 11, wherein at least one of said light sources (3, 31, 32) comprises at least one LED (301) for emitting warm-white color temperature light and at least one LED (302) for emitting infrared light.

13. The polarized light emitting medical device according to any of claims 1 to 12, wherein said electronic control unit (2) and a driving circuit (8) of said polarized light emitting arrangement are fitted on a printed circuit board (7).

14. The polarized light emitting medical device according to any of claims 1 to 13, wherein said electronic control unit (2) comprises control panel/s/ (701, 702) for light source control.

15. The polarized light emitting medical device according to any of claims 1 to 14, wherein said polarized light emitting treatment console/s/ (6, 61, 62) is/are equipped with overheating sensor/s/ (5, 51, 52) which are signal-connected with the electronic control unit (2).

16. The polarized light emitting medical device according to any of claims 6 to 15, wherein said polarized light emitting treatment console/s/ (6, 61, 62) feature/s/ an oval window (30) formed for said polarizer optical unit (4, 41, 42) at the distal end portion of said polarized light emitting treatment console/s/ (6, 61, 62), the longer axis (AL) of said oval window (30) being parallel to the longitudinal direction of the respective polarized light emitting console (6, 61, 62), and said polarizer optical unit (4, 41, 42) is positioned in said oval window (30) to define the direction of polarization (DP) parallel to the longer axis (AL) of the oval window (30).

## Patentansprüche

1. Polarisiertes Licht emittierende medizinische Vorrichtung, insbesondere zur Vorbeugung und Behandlung von gingivalen und periodontalen Erkrankungen, mit einem Gehäuse (1), das als Griff der medizinischen Vorrichtung ausgebildet ist, das eine mit einer polarisierten lichtemittierenden Anordnung in steuernder Verbindung stehende elektronische Steuereinheit (2) umfasst, wobei die polarisiertes Licht emittierende Anordnung mindestens eine mit der elektronischen Steuereinheit (2) verbundene Lichtquelle (3, 31, 32), und eine optisch mit der Lichtquelle (3, 31, 32) verbundene optische Polarisatoreinheit (4, 41, 42) umfasst, um aus den von der Lichtquelle (3, 31, 32) emittierten Lichtstrahlen einen polarisierten Lichtstrahl (P) zu bilden, wobei die polarisiertes Licht emittierende Anordnung mindestens eine polarisiertes Licht emittierende Behandlungskonsole (6, 61, 62) umfasst, die mit dem Gehäuse (1) verbunden ist und die Lichtquelle (3, 31, 32) und die mit dieser optisch verbundene optische Polarisatoreinheit (4, 41, 42) aufnimmt, **dadurch gekennzeichnet, dass** die mindestens eine polarisiertes Licht emittierende Behandlungskonsole (6, 61, 62) ein ovales Fenster (30) ist, das für die optische Polarisatoreinheit (4, 41, 42) am distalen Endabschnitt der polarisiertes Licht emittierenden Polarisatoreinheit(en) (6, 61, 62) ausgebildet ist, wobei die längere Achse (AL) des ovalen Fensters (30) parallel zu der Längsrichtung der jeweiligen polarisiertes Licht emittierenden Polarisationskonsole (6, 61, 62) verläuft, und die optische Polarisatoreinheit (4, 41, 42) in dem ovalen Fenster (30) positioniert ist, um die Polarisationsrichtung (DP) parallel zu der längeren Achse (AL) des ovalen Fensters (30) zu bestimmen.

2. Die polarisiertes Licht emittierende medizinische Vorrichtung nach Anspruch 1, **wobei** die mindestens eine, polarisiertes Licht emittierende Behandlungskonsole (6, 61, 62) eine zeltenförmige Komponente ist, wobei die Lichtquelle (3, 31, 32) und die damit optisch verbundene optische Polarisatoreinheit (4, 41, 42) im distalen Endabschnitt der zeltenförmigen Behandlungskonsole (6, 61, 62) angeordnet sind.

3. Die polarisiertes Licht emittierende medizinische Vorrichtung nach einem der Ansprüche 1 oder 2, **wobei** die mindestens eine, polarisiertes Licht emittierende Behandlungskonsole (6, 61, 62) eine zeltenförmige gebogene geometrische Gestaltung aufweist, die in Richtung der Emission von polarisiertem Licht (P) relativ zur Längsachse (t) des Gehäuses (1) nach innen gebogen ist.

4. Die polarisiertes Licht emittierende medizinische Vorrichtung nach einem der Ansprüche 2 oder 3, **wobei** sie zwei zeltenförmige, polarisiertes Licht emittierende Behandlungskonsolen (61, 62) aufweist, die jeweils mit den entgegengesetzten Enden des Gehäuses (1) verbunden sind, wobei beide zeltenförmigen polarisiertes Licht emittierende Behandlungskonsolen (61, 62) unterschiedliche geometrische Gestaltungen aufweisen.

5. Die polarisiertes Licht emittierende medizinische Vorrichtung nach Anspruch 4, **wobei** die eine der zeltenförmigen, polarisiertes Licht emittierenden Behandlungskonsolen (61), die mit dem Gehäuse (1) verbunden ist, eine in einem Winkel (α₁ ) 22 innerhalb eines Bereichs von 10° bis 90° in der Richtung der Emission des polarisierten Lichts (P) relativ zu der Längsachse (t) des Gehäuses (1) nach innen gebogene geometrische Gestaltung aufweist, während die andere zeltenförmige, polarisiertes Licht emittierende Behandlungskonsole (62) eine in einem Winkel (α₂) innerhalb eines Bereichs von 90° bis 170° in der Richtung der Emission des polarisierten Lichts (P) relativ zu der Längsachse (t) des Gehäuses (1) nach innen gebogene geometrische Gestaltung aufweist.

6. Polarisiertes Licht emittierende medizinische Vorrichtung, insbesondere zur Vorbeugung und Behandlung von gingivalen und periodontalen Erkrankungen, mit einem Gehäuse (1), das als Griff der medizinischen Vorrichtung ausgebildet ist, das eine mit einer polarisierten lichtemittierenden Anordnung in steuernder Verbindung stehende elektronische Steuereinheit (2) umfasst, wobei die polarisiertes Licht emittierende Anordnung mindestens eine mit der elektronischen Steuereinheit (2) verbundene Lichtquelle (3, 31, 32), und eine optisch mit der Lichtquelle (3, 31, 32) verbundene optische Polarisatoreinheit (4, 41, 42) umfasst, um aus den von der Lichtquelle (3, 31, 32) emittierten Lichtstrahlen einen polarisierten Lichtstrahl (P) zu bilden, wobei die polarisiertes Licht emittierende Anordnung mindestens eine polarisiertes Licht emittierende Behandlungskonsole (6, 61, 62) umfasst, die mit dem Gehäuse (1) verbunden ist und die Lichtquelle (3, 31, 32) und die mit dieser optisch verbundene optische Polarisatoreinheit (4, 41, 42) aufnimmt, **dadurch gekennzeichnet, dass** eine der zeltenförmigen, polarisiertes Licht emittierenden Behandlungskonsolen (61), die mit dem Gehäuse (1) verbunden ist, eine in einem Winkel (α₁) innerhalb eines Bereichs von 15° bis 60° in der Richtung der Emission des polarisierten Lichts (P) relativ zu der Längsachse (t) des Gehäuses (1) nach innen gebogene geometrische Gestaltung aufweist, während die andere zeltenförmige, polarisiertes Licht emittierende Behandlungskonsole (62) eine in einem Winkel (α₂) innerhalb eines Bereichs von 90° bis 135° in der Richtung der Emission des polarisierten Lichts (P) relativ zu der Längsachse (t) des Gehäuses (1) nach innen gebogene geometrische Gestaltung aufweist.

7. Die polarisiertes Licht emittierende medizinische Vorrichtung nach einem der Ansprüche 1 bis 6, **wobei** die mindestens eine, polarisiertes Licht emittierende Behandlungskonsole (6, 61, 62) eine festhaltend biegbare zeltenförmige Fassung aufweist.

8. Die polarisiertes Licht emittierende medizinische Vorrichtung nach einem der Ansprüche 1 bis 7, **wobei** die mindestens eine, polarisiertes Licht emittierende Behandlungskonsole (6, 61, 62) ein austauschbares Bauteil ist, das durch eine lösbare Verbindung mit dem Gehäuse (1) verbunden ist.

9. Die polarisiertes Licht emittierende medizinische Vorrichtung nach einem der Ansprüche 1 bis 6, **wobei** die mindesten eine, eine zeltenförmige Fassung aufweisende polarisiertes Licht emittierende Behandlungskonsole (61, 62) ein monolithisches Strukturstück mit dem Gehäuse (1) bildet.

10. Die polarisiertes Licht emittierende medizinische Vorrichtung nach einem der Ansprüche 1 bis 9, **wobei** das als Griff der medizinischen Vorrichtung ausgebildete Gehäuse (1) mit von außen betätigbaren Steuerknöpfen (13, 14) ausgestattet ist, die mit der elektronischen Steuereinheit (2) verbunden sind.

11. Die polarisiertes Licht emittierende medizinische Vorrichtung nach einem der Ansprüche 1 bis 10, **wobei** die Lichtquelle oder die Lichtquellen (3, 31, 32) jeweils aus einer Halogenlichtquelle/-en/und/oder Leuchtdioden (LEDs) gebildet ist/sind.

12. Die polarisiertes Licht emittierende medizinische Vorrichtung nach einem der Ansprüche 1 bis 11, **wobei** mindestens eine der Lichtquellen (3, 31, 32) mindestens eine LED (301) zum Emittieren von warmweißem Farbtemperaturlicht und mindestens eine LED (302) zum Emittieren von Infrarotlicht umfasst.

13. Die polarisiertes Licht emittierende medizinische Vorrichtung nach einem der Ansprüche 1 bis 12, **wobei** die elektronische Steuereinheit (2) und eine Treiberschaltung (8) der Anordnung mit polarisiertem Licht auf einer Leiterplatte (7) angebracht sind.

14. Die polarisiertes Licht emittierende medizinische Vorrichtung nach einem der Ansprüche 1 bis 13, **wobei** die elektronische Steuereinheit (2) Bedienfeld(er) (701, 702) zur Lichtquellensteuerung umfasst.

15. Die polarisiertes Licht emittierende medizinische Vorrichtung nach einem der Ansprüche 1 bis 14, **wobei** die ein polarisiertes Licht emittierende Behandlungskonsole(n) (6, 61, 62) mit, mit der elektronischen Steuereinheit (2) signalverbunden Überhitzungssensor(en) (5, 51, 52) ausgestattet ist/sind.

16. Die polarisiertes Licht emittierende medizinische Vorrichtung nach einem der Ansprüche 6 bis 15, **wobei** die mindestens eine polarisiertes Licht emittierende Behandlungskonsole (6, 61, 62) ein ovales Fenster (30) ist, das für die optische Polarisatoreinheit (4, 41, 42) am distalen Endabschnitt der polarisiertes Licht emittierenden Polarisatoreinheit(en) (6, 61, 62) ausgebildet ist, wobei die längere Achse (AL) des ovalen Fensters (30) parallel zu der Längsrichtung der jeweiligen polarisiertes Licht emittierenden Polarisationskonsole (6, 61, 62) verläuft, und die optische Polarisatoreinheit (4, 41, 42) in dem ovalen Fenster (30) positioniert ist, um die Polarisationsrichtung (DP) parallel zu der längeren Achse (AL) des ovalen Fensters (30) zu bestimmen.

## Revendications

1. Dispositif médical émetteur de lumière polarisée, en particulier pour la prévention et le traitement de maladies gingivales et parodontales, avec un boîtier (1) formé en tant que poignée du dispositif médical, dans lequel il y a une unité de commande électronique (2) qui est connectée par un signal de commande à un agencement émetteur de lumière polarisée, ledit agencement émetteur de lumière polarisée comprenant au moins une source de lumière (3, 31, 32) connectée à ladite unité de commande électronique (2) et une unité optique de polariseur (4, 41, 42) connectée optiquement à ladite source de lumière (3, 31, 32) pour former un faisceau de lumière polarisée (P) à partir des rayons de lumière émis par ladite source de lumière (3, 31, 32), ledit agencement émetteur de lumière polarisée comprenant au moins une console de traitement émetteur de lumière polarisée (6, 61, 62) connectée audit boîtier (1) et incorporant ladite source de lumière (3, 31, 32) et ladite unité optique de polariseur (4, 41, 42) connectée optiquement à celle-ci, **caractérisé en ce que** ladite/lesdites console(s) de traitement émetteur(s) de lumière polarisée (6, 61, 62) comportent une fenêtre ovale (30) formée pour ladite unité optique de polariseur (4, 41, 42) au niveau de la partie d'extrémité distale de ladite/lesdites console(s) de traitement émetteur(s) de lumière polarisée (6, 61, 62), l'axe plus long (AL) de ladite fenêtre ovale (30) étant parallèle à la direction longitudinale de la console émetteur de lumière polarisée respective (6, 61, 62), et ladite unité optique de polariseur (4, 41, 42) est positionnée dans ladite fenêtre ovale (30) pour définir la direction de polarisation (DP) parallèle à l'axe plus long (AL) de la fenêtre ovale (30).

2. Dispositif médical émetteur de lumière polarisée selon la revendication 1, dans lequel ladite au moins une console de traitement émetteur de lumière polarisée (6, 61, 62) est un composant en forme de tentacule, dans lequel ladite source de lumière (3, 31, 32) et ladite unité optique de polariseur (4, 41, 42) optiquement connectée à celle-ci sont agencées dans la partie d'extrémité distale de ladite console de traitement en forme de tentacule (6, 61, 62).

3. Dispositif médical émetteur de lumière polarisé selon l'une quelconque des revendications 1 ou 2, dans lequel ladite au moins une console de traitement émetteur de lumière polarisée (6, 61, 62) a une conception géométrique fléchie de type tentacule, fléchie vers l'intérieur dans la direction de l'émission de lumière polarisée (P) par rapport à l'axe longitudinal (t) du boîtier (1).

4. Dispositif médical émetteur de lumière polarisée selon l'une quelconque des revendications 2 ou 3, dans lequel il a deux consoles de traitement émetteur(s) de lumière polarisée en forme de tentacule (61, 62) chacune reliées aux extrémités opposées dudit boîtier (1), dans lequel les deux consoles de traitement émetteur(s) de lumière polarisée en forme de tentacule (61, 62) ont des conceptions géométriques différentes.

5. Dispositif médical émetteur de lumière polarisée selon la revendication 4, dans lequel l'une des consoles de traitement émetteur de lumière polarisée en forme de tentacule (61) reliée au boîtier (1) a une conception géométrique d'être fléchie vers l'intérieur à un angle (α₁) dans une plage de 10° à 90° dans la direction de l'émission de la lumière polarisée (P) par rapport à l'axe longitudinal (t) du boîtier (1), tandis que l'autre console de traitement émetteur de lumière polarisée en forme de tentacule (62) a une conception géométrique d'être fléchie vers l'intérieur à un angle (α₂) dans une plage de 90° à 170° dans la direction de l'émission de la lumière polarisée (P) par rapport à l'axe longitudinal (t) du boîtier (1).

6. Dispositif médical émetteur de lumière polarisée, en particulier pour la prévention et le traitement de maladies gingivales et parodontales, avec un boîtier (1) formé en tant que poignée du dispositif médical, dans lequel il y a une unité de commande électronique (2) qui est connectée par un signal de commande à un agencement émetteur de lumière polarisée, ledit agencement émetteur de lumière polarisée comprenant au moins une source de lumière (31, 32) connectée à ladite unité de commande électronique (2) et une unité optique de polariseur (41, 42) connectée optiquement à ladite source de lumière (31, 32) pour former un faisceau de lumière polarisée (P) à partir des rayons de lumière émis par ladite source de lumière (31, 32), ledit agencement émetteur de lumière polarisée comprenant deux consoles de traitement émetteur(s) de lumière polarisée en forme de tentacule (61, 62) chacune reliées aux extrémités opposées dudit boîtier (1) et incorporant ladite source de lumière (31, 32) et ladite unité optique de polariseur (41, 42) connectée optiquement à ceux-ci, **caractérisé en ce que** l'une des consoles de traitement émetteur de lumière polarisée en forme de tentacule (61) reliée au boîtier (1) a une conception géométrique d'être fléchie vers l'intérieur à un angle (α₁) dans une plage de 15° à 60° dans la direction de l'émission de la lumière polarisée (P) par rapport à l'axe longitudinal (t) du boîtier (1), tandis que l'autre console de traitement émetteur de lumière polarisée en forme de tentacule (62) a une conception géométrique d'être fléchie vers l'intérieur à un angle (α₂) dans une plage de 90° à 135° dans la direction de l'émission de la lumière polarisée (P) par rapport à l'axe longitudinal (t) du boîtier (1).

7. Dispositif médical émetteur de lumière polarisée selon l'une quelconque des revendications 1 à 6, dans lequel ladite au moins une console de traitement émetteur de lumière polarisée (6, 61, 62) a une douille en forme de tentacule pliable de façon rémanente.

8. Dispositif médical émetteur de lumière polarisé selon l'une quelconque des revendications 1 à 7, dans lequel ladite au moins une console de traitement émetteur de lumière polarisée (6, 61, 62) est un composant interchangeable relié par une liaison libérable au boîtier (1).

9. Dispositif médical émetteur de lumière polarisée selon l'une quelconque des revendications 1 à 6, dans lequel ladite/lesdites console(s) de traitement émetteur(s) de lumière polarisée (61, 62) a/ont une/des douille(s) en forme de tentacule, et forme(nt) une pièce structurale monolithique avec le boîtier (1).

10. Dispositif médical émetteur de lumière polarisé selon l'une quelconque des revendications 1 à 9, dans lequel ledit boîtier (1) formé en tant que poignée du dispositif médical est équipé de boutons de commande actionnables externes (13, 14) qui sont connectés à ladite unité de commande électronique (2).

11. Dispositif médical émetteur de lumière polarisé selon l'une quelconque des revendications 1 à 10, dans lequel ladite ou lesdites source(s) de lumière (3, 31, 32), respectivement, est/sont constituée(s) par une/des source(s) lumineuse(s) halogène(s) et/ou des diodes électroluminescentes (DELs).

12. Dispositif médical émetteur de lumière polarisé selon l'une quelconque des revendications 1 à 11, dans lequel au moins l'une desdites sources de lumière (3, 31, 32) comprend au moins une DEL (301) pour émettre une lumière de température de couleur blanche chaude et au moins une DEL (302) pour émettre une lumière infrarouge.

13. Dispositif médical émetteur de lumière polarisé selon l'une quelconque des revendications 1 à 12, dans lequel ladite unité de commande électronique (2) et un circuit d'attaque (8) dudit agencement émetteur de lumière polarisé sont montés sur une carte de circuit imprimé (7).

14. Dispositif médical émetteur de lumière polarisé selon l'une quelconque des revendications 1 à 13, dans lequel ladite unité de commande électronique (2) comprend un/des panneau(x) de commande (701, 702) pour une commande de source de lumière.

15. Dispositif médical émetteur de lumière polarisée selon l'une quelconque des revendications 1 à 14, dans lequel ladite/lesdites console(s) de traitement émetteur(s) de lumière polarisée (6, 61, 62) est/sont équipée(s) d'un/des capteur(s) de surchauffe (5, 51, 52) qui sont connectés en signal avec l'unité de commande électronique (2).

16. Dispositif médical émetteur de lumière polarisé selon l'une quelconque des revendications 6 à 15, dans lequel ladite/lesdites console(s) de traitement émetteur(s) de lumière polarisée (6, 61, 62) comportent une fenêtre ovale (30) formée pour ladite unité optique de polariseur (4, 41, 42) au niveau de la partie d'extrémité distale de ladite/lesdites console(s) de traitement émetteur(s) de lumière polarisée (6, 61, 62), l'axe plus long (AL) de ladite fenêtre ovale (30) étant parallèle à la direction longitudinale de la console émetteur de lumière polarisée respective (6, 61, 62), et ladite unité optique de polariseur (4, 41, 42) est positionnée dans ladite fenêtre ovale (30) pour définir la direction de polarisation (DP) parallèle à l'axe plus long (AL) de la fenêtre ovale (30).
